# EUROPEAN PATENT SPECIFICATION

(11) **EP 4 241 716 B1**
(45) Date of publication and mention of the grant of the patent: **27.08.2025**
(21) Application number: 23160113.9
(22) Date of filing: 06.03.2023
(51) Int. Cl.: A61B 34/20, A61B 90/00, A61B 34/10

(54) **TECHNIQUE FOR DETERMINING A PATIENT-SPECIFIC MARKER ARRANGEMENT FOR A TRACKER OF A SURGICAL TRACKING SYSTEM**
TECHNIK ZUR BESTIMMUNG EINER PATIENTENSPEZIFISCHEN MARKERANORDNUNG FÜR EINEN TRACKER EINES CHIRURGISCHEN TRACKINGSYSTEMS
TECHNIQUE DE DÉTERMINATION D'UN AGENCEMENT DE MARQUEUR SPÉCIFIQUE À UN PATIENT POUR UN DISPOSITIF DE SUIVI D'UN SYSTÈME DE SUIVI CHIRURGICAL

(30) Priority: 08.03.2022 US 202217689382
(43) Date of publication of application: 13.09.2023
(73) Proprietor: Stryker European Operations Limited, Carrigtwohill, Co. Cork T45 HX08 (IE)
(72) Inventor: FRANITZA, Florian, 79238 Ehrenkirchen (DE); GHANAM, Fadi, 79227 Schallstadt (DE)
(74) Representative: Röthinger, Rainer

(56) References cited:
- EP-B1- 3 076 892
- US-A1- 2006 212 044
- US-A1- 2010 137 712

## Description

### Technical Field

The present disclosure generally relates to the tracking of objects, for example to patient tracking in a surgical navigation context. In particular, a method for determining a patient-specific marker arrangement for a tracker of a surgical tracking system is presented. Also presented are a computer program product, an apparatus for determining a patient-specific marker arrangement, and a system comprising the apparatus and a manufacturing device configured to manufacture the tracker with the marker arrangement.

### Background

Surgical tracking systems are configured to track a surgical object such as a surgical instrument or a patient during a surgical procedure. To this end, one or more trackers are attached to the object that is to be tracked. Each tracker comprises one or more markers configured to be detected by a tracking sensor of the tracking system. Optical tracking systems, for example, use a tracking camera to capture image data representative of optically detectable markers. Based on positions of the markers in the image data, information about one or both of a position and an orientation of the tracker and, thus, of the tracked object can be determined.

The positions of the markers of a given tracker define a marker arrangement. Information indicative of these positions is typically required for the tracking system to identify the markers (and, thus, the tracker) in the image data.

Conventional trackers are mass-produced at high precision to ensure that they all have an identical marker arrangement. Therefore, all the trackers of a given tracker type have exactly the same relative positions between the markers. The tracking system thus only requires knowledge about these relative positions for a given tracker type, but not for each individual tracker. It has, however, been found that such pre-defined tracker types are not suitable for all use cases. For example, they may block a surgeon's activities or result in loss-of-sight issues.

In a surgical tracking context, the tracking camera requires a continuous line-of-sight towards the tracker that has previously been registered relative to a camera coordinate system. In case the line-of-sight is lost, the tracker can no longer be tracked and procedures relying on the tracker, such a navigation procedures, have to be paused or aborted.

A particular object may carry multiple trackers to allow for a continuous tracking even if the tracking camera loses its line-of-sight to one of the trackers. However, using multiple trackers may hinder a surgeon, i.e., may block surgical access or the surgeon's line-of-sight to an anatomical region of interest.

A technique for planning a tracker location to reduce a line-of-sight loss is discussed in WO 2014/159350 A1. The tracker location is determined based on at least one of pre- and inter-operative information regarding predetermined or tracked positions of objects located in an operating room.

Document US 2006/0212044 A1 discloses devices, methods and systems for frameless guidance of image-based medical procedures such as stereotactic radiotherapy and surgery. Devices including custom fitting subject-specific articles include contoured surfaces that provide spatial reference to the location of target regions within the subject. The devices are attached exclusive of fasteners anchored to the tissue of the subject and reside on the front of a head of the subject when attached. The devices and articles can be fabricated using computer-directed fabrication technology

Document US 2010/0137712 A1 discloses systems and methods for stabilizing a target location within a human body. The system provides a tissue anchor for holding a tissue mass within a human body. The tissue anchor may include a lead; a tissue fastener coupled to the lead; and a marker which can be detected by a position detection system to facilitate placement of the anchor. The tissue anchor can be used to help stabilize tissue in a surgical procedure, e.g., in excising a lesion in amorphous, pliable tissue (e.g., breast tissue) or other body parts.

### Summary

There is a need for a marker arrangement for tracker that avoids one or more of the above or other problems.

According to the present disclosure, there is provided a computer-implemented method, a computer program product, an apparatus and a system according to the independent claims. Developments are set forth in the dependent claims.

According to a first aspect, a method for determining a patient-specific marker arrangement for a tracker of a surgical tracking system is provided. The marker arrangement is defined by marker positions. The method comprises receiving three-dimensional image data of a patient, obtaining, based on the three-dimensional image data, planning data for a surgical intervention, and determining the marker arrangement for the tracker based at least on the obtained planning data.

The marker arrangement may be defined in various ways. In some cases, the marker arrangement is defined by relative (e.g., Euclidean distances) between the marker positions. In other cases, the marker arrangement is defined by marker positions in a tracker coordinate system.

The three-dimensional image data may have been acquired using a three-dimensional medical imaging technology, such as computed tomography (CT) imaging, magneto-resonance imaging (MRI), or a two-dimensional medical imaging technology that permits a three-dimensional image reconstruction (e.g., by taking two-dimensional X-ray images from different orientations of a C-arm).

The marker arrangement may be a (planar or non-planar) two-dimensional or a three-dimensional arrangement. In certain variants, the method may comprise generating manufacturing instructions for manufacturing the marker arrangement as determined based at least on the obtained planning data. Thus, a customized tracker comprising an optimized marker arrangement may be manufactured (e.g., optimized from an obstruction perspective). The manufacturing instructions may comprise at least one of printing instructions and instructions for additive manufacturing (e.g., for three-dimensional printing). The manufacturing instructions may be machine-readable instructions (e.g., to control a printer or additive manufacturing device) or instructions addressed to a user (e.g., how to cut out the marker arrangement from a larger sheet on which multiple markers are printed or otherwise realized).

An exemplary manufacturing approach may comprise the steps of providing a substrate with at least one reflective surface configured to reflect electromagnetic radiation, and printing an absorbent layer configured to absorb electromagnetic radiation over the substrate in such a way that the absorbent layer covers less than the entire reflective surface. The substrate and the absorbent layer may form a layer stack. The absorbent layer may be printed to realize the marker arrangement as determined based at least on the obtained planning data. The marker arrangement may additionally be determined based on at least one of a surgery setup and tracker design rules (e.g., regarding a minimum and/or maximum number of markers, minimum and/or maximum distances between markers, etc.). The marker arrangement may take the form of a pattern.

At least one of the planning data (e.g., in the form of a surgery plan) and the surgery setup may define at least one of: a field of view of a signal detector (e.g., a tracking camera), a position and/or orientation of a patient or a body part of the patient, and a position and/or orientation of a surgeon. The marker arrangement may be dimensioned to scale with the viewing angle, or a dimension (e.g., size) of the patient. The marker arrangement may be printed to fit onto a surface of the patient covered by the field of view of the tracking camera and not blocked by the surgeon.

The planning data (e.g., in the form of a surgery plan) may define at least one of an area of interest of the patient, a dedicated surgical procedure, and a trajectory of a surgical instrument. The marker arrangement may be printed to fit onto a surface of a body part that includes the area of interest. The marker arrangement may be printed to fit onto a surface of the patient that is not covered by (e.g., intersects with) the trajectory. The surface of the patient may be covered by the trajectory if a projection of the trajectory perpendicular the patient surface or along the field of view coincides with said surface of the patient.

The tracker design rules may define at least one marker or marker arrangement where the absorbent layer has an opening that does not cover the reflective surface. The tracker design rules may define at least one of a size and a shape of the at least one marker or the marker arrangement. The tracker design rules may define a minimum distance between markers. The minimum distance between markers may depend on a distance of the tracker to the tracking camera (e.g., the minimum marker distance may increase with increasing distance to the tracking camera, and vice versa). Additionally, or in the alternative, the minimum distance between markers may depend on a distance of the tracker to the anatomy that is to be tracked (e.g., the minimum marker distance may decrease with decreasing distance to the anatomy to be tracked, and vice versa).

In some variants, the method may comprise determining a surface of the patient based on the three-dimensional image data. Conventional image processing techniques such as thresholding can be used in this regard (e.g., thresholding of Hounsfield values obtained by a CT scan). In such an implementation, the marker arrangement may be determined also based on the determined surface of the patient. Determining the marker arrangement also based on the patient surface may comprise virtually arranging one or more markers on the determined surface of the patient. The marker arrangement may be determined based on the virtually arranged markers (e.g., in accordance with their virtually arranged pattern and/or distances).

Taking the surface of a patient into account may facilitate positioning of the tracker on the patient. As an example, the marker arrangement may be determined to not include markers on a protruding body part of the patient (e.g., a patient' nose) as derived from the determined surface. As a result, the tracker may be configured to have an opening or indentation to accommodate the protruding body part. In another example, the marker arrangement may be determined taking into account a surface curvature at the position where the tracker is to be placed on the patient.

In some variants, the planning data may be indicative of an anatomical region of interest of the patient. Determining the marker arrangement may comprise virtually arranging one or more markers relative to the anatomical region of interest. The markers may, for example, be arranged to surround the region of interest. The marker arrangement may be determined based on the virtually arranged markers (e.g., in accordance with their virtually arranged pattern and/or distances). Based at least in part on the region of interest, a size of the tracker and thus at least one of a size, a number and relative positions of the markers to each other may be determined. For example, the further the tracker is away from the region of interest, the larger it must be designed in size to achieve a desired accuracy. The desired accuracy may be predefined, e.g., according to a specific medical field and use-case.

In some variants, the planning data may be indicative of a planned trajectory of a surgical instrument. Determining the marker arrangement may comprise virtually arranging one or more markers relative to the planned trajectory. The marker arrangement may be determined based on the virtually arranged markers. For example, the marker arrangement may be determined in order to prevent collision of the tracker, i.e., a tracker's carrier substrate and the markers, with a surgical instrument moved along the planned trajectory during surgery. Determining the marker arrangement may comprise taking into account a margin for correction of the planned trajectory of the surgical instrument. The margin for correction may be predefined, e.g., according to a specific medical field and use-case.

In some variants, the planning data may be indicative of a planned incision.

Determining the marker arrangement may comprise virtually arranging one or more markers relative to the planned incision. The marker arrangement may be determined based on the virtually arranged markers. The planned incision may be taking into account to prevent complicating access of a surgeon to the anatomical region of interest. For example, the markers may have a predefined minimum distance to the planned incision. Determining the marker arrangement may comprise taking into account a margin for correction of the planned incision.

The planning data are indicative of a planned position of a signal generator or signal detector of the surgical tracking system relative to a patient anatomy represented in the three-dimensional image data. Such a signal generator or signal detector may be provided for generating or detecting, respectively, an optical signal (e.g., in the infrared or visible spectrum). Taking into account the planned position, a region of the three-dimensional image data that has a line-of-sight to the signal generator or signal detector may be determined based on the planning data. The marker arrangement may then be determined based on the determined region of the three-dimensional image data.

In some variants, the tracker may have a substrate supporting the marker arrangement, and the method may comprise determining a shape of the substrate based on the planning data. The manufacturing instructions described above may comprise trimming instructions, e.g., instructions on using scissors or perforations, in order to obtain a custom-shaped tracker from the substrate. The trimming instructions may comprise trimming markings printed on the tracker substrate, e.g., on a layer stack, that indicate where the substrate is to be trimmed. Alternatively or additionally, the trimming instructions may be provided on a display, e.g., in form of an image of the substrate, e.g., the layer stack, with trimming markings or an animation of how to trim the substrate. The trimming instructions may be determined based on at least one of a surgery setup, the planning data, and tracker design rules as described herein. The marker arrangement may be determined based on the trimmed tracker (e.g., such that it fits on the trimmed tracker).

In some variants, the three-dimensional image data may have been captured by an imaging apparatus (e.g., a medical imaging apparatus configured to image a patient anatomy). In such a scenario, the method may comprise receiving positional data indicative of a position of the imaging apparatus when capturing the three-dimensional image data, wherein the marker arrangement is determined based on the positional data. The position of the imaging apparatus may be determined in a coordinate system of an operating room, i.e., relative to other objects in the operating room like the signal generator or signal detector of the surgical tracking system.

In some variants, determining the marker arrangement may take into account a criterion related to a minimum distance between markers. The minimum distance between markers may depend on a distance of the tracker to the signal detector, e.g., a tracking camera (e.g., the minimum marker distance may increase with increasing distance to the tracking camera, and vice versa). Additionally, or in the alternative, the minimum distance between markers may depend on a distance of the tracker to the anatomy that is to be tracked (e.g., the minimum marker distance may decrease with decreasing distance to the anatomy to be tracked, and vice versa).

According to a second aspect, a computer program product, comprising instructions that, when executed on at least one processor, cause the at least one processor to carry out any of the method steps described herein. The computer program product may be stored on a computer-readable recording medium.

According to a third aspect, an apparatus for determining a patient-specific marker arrangement for a tracker of a surgical tracking system is provided. The marker arrangement is defined by marker positions. The apparatus is configured to receive three-dimensional image data of a patient, and wherein the planning data also are indicative of a planned position of a signal generator or signal detector of the surgical tracking system relative to a patient anatomy represented in the three-dimensional image data; to obtain, based on the three-dimensional image data, planning data for a surgical intervention; and to determine the marker arrangement for the tracker based at least on the obtained planning data.

In some variants, the apparatus may be configured to perform any of the method steps described herein.

According to a fourth aspect, a system is provided. The system comprises the apparatus for determining a patient-specific marker arrangement described herein and a manufacturing device configured to manufacture a tracker with the determined marker arrangement.

### Brief Description of the Drawings

Further details, advantages and aspects of the present disclosure will become apparent from the following embodiments taken in conjunction with the drawings, wherein:
- Fig. 1: schematically illustrates a planned surgical intervention;
- Fig. 2: shows a flow diagram of a method for determining a marker arrangement for a tracker of a surgical tracking system;
- Fig. 3A: schematically shows three-dimensional image data of a patient;
- Fig. 3B: schematically shows planned trajectories of a surgical instrument relative to the three-dimensional image data of Fig. 3A;
- Fig. 3C: schematically shows a tracker virtually superimposed on the three-dimensional image data of Fig. 3B;
- Fig. 4A: schematically shows three-dimensional image data of the patient and a planned position of a surgeon relative to the three-dimensional image data;
- Fig. 4B: schematically shows a tracker superimposed on the three-dimensional image data of Fig. 4A;
- Fig. 5A: schematically shows three-dimensional image data of the patient and a planned position and orientation of a signal detector of the surgical tracking system relative to the three-dimensional image data;
- Fig. 5B: schematically shows the three-dimensional image data of Fig. 5A with an indicated region suitable for arranging markers therein;
- Fig. 5C: schematically shows a tracker superimposed on the indicated region of Fig. 5B;
- Fig. 6A: schematically shows three-dimensional image data of the patient with planned positions of the surgeon and the signal detector;
- Fig. 6B: schematically shows the three-dimensional image data of Fig. 6A with an indicated region suitable for arranging markers therein;
- Fig. 6C: schematically shows a tracker superimposed on the indicated region of Fig. 6B;
- Fig. 7A: schematically shows three-dimensional image data of a patient with an indicated region suitable for arranging markers therein;
- Fig. 7B: schematically shows the three-dimensional image data of Fig. 7A, with the indicated region being restricted based on planned trajectories of the surgical instrument;
- Fig. 7C: schematically shows the three-dimensional image data of Fig. 7B, with the indicated region being further restricted based on the planned position of the surgeon relative to the three-dimensional image data;
- Fig. 7D: schematically shows the three-dimensional image data of Fig. 7C, with the indicated region being further restricted based on the planned position and orientation of the signal detector;
- Fig. 7E: schematically shows a tracker superimposed on the indicated region of Fig. 7D; and
- Fig. 7F: schematically shows a display of manufacturing instructions for trimming the tracker of Fig. 7E from a tracker substrate.

### Detailed Description

In the following description of exemplary embodiments, aspects in the context of determining a patient-specific marker arrangement for a tracker of a surgical tracking system are presented. The tracker with the patient-specific marker arrangement may be configured to be detectable by any tracking technology, in particular involving emission or reflection of electromagnetic radiation (e.g., in the visible or infrared spectrum). The same reference numerals are used to denote the same or similar features.

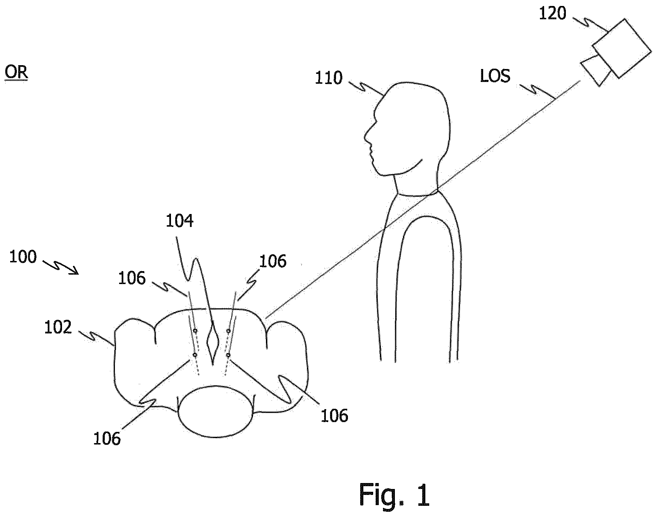

Fig. 1 schematically illustrates the planning of a surgical intervention. Details of the planned surgical intervention are defined in planning data.

In more detail, Fig. 1 illustrates a visualization, or representation, of three-dimensional image data 102 of a patient 100. The three-dimensional image data 102 have been captured using a medical imaging technology, e.g., using an x-ray apparatus, a CT apparatus or an MRI apparatus (not shown in Fig. 1).

Fig. 1 indicates a position of a planned incision 104 and several planned trajectories 106 of one or more surgical instruments (not shown in Fig. 1) relative to the three-dimensional image data 102. Corresponding positional and/or orientational information may be comprised by the planning data. Still further, the planning data may also indicate a position and orientation of a surgeon 110 and of a signal detector 120, e.g., a tracking camera, of the surgical tracking system relative to the three-dimensional image data 102. The planning data may be generated in an automated manner or responsive to a user input (e.g., by a surgeon). It will be apparent that the scenario of Fig. 1 is only illustrative. As an example, the planning data may only be indicative of a single planned trajectory or incision, optionally in combination with a position of at least one of the surgeon 110 and the signal detector 120.

One or more coordinate systems may be defined for one or more of the three-dimensional image data 102, the surgeon 110 and the surgical tracking system (e.g., relative to the tracking camera 120). In some variants, the planning data is represented in such one or more coordinate systems. In particular, the planning data may be represented in a coordinate system of the three-dimensional image data (i.e., the image coordinate system). The coordinate system of the three-dimensional image data 102 may be defined by the medical imaging apparatus capturing the three-dimensional image data 102. The planned position of the surgeon 110 may also be indicated in the coordinate system of the three-dimensional image data 102. Alternatively, the surgeon 110 may be tracked by the surgical tracking system or another tracking system with a known position relative to the surgical tracking system, and the position of the surgeon 110 may be determined within the coordinate system of the surgical tracking system. The coordinate system of the surgical tracking system may be defined relative to a signal generator or the signal detector 120 of the surgical tracking system.

The individual coordinate systems may be registered with each other or with a global coordinate system, e.g., a coordinate system of an operating room, OR, in which the surgical intervention will take place. The OR may accommodate the patient 100, the surgeon 110, and the surgical tracking system. After one or more coordinate system registrations, the planning data (e.g., in the image coordinate system) may include the positions and/or orientations of the surgeon 110 and the signal detector 120 relative to the position of the three-dimensional image data 102 and relative to each other. In such or other variants, the planning data may indicate one or more of the planned incision 104 and at least one planned trajectory 106 of one or more surgical instruments relative to the position of the three-dimensional image data 102.

The surgical tracking system may comprise one or more trackers, the signal detector 120 and, optionally, the signal generator. The one or more trackers may be attached to the patient 100, and, optionally, to at least one of the surgeon 120 and the one or more surgical instruments. The surgical tracking system may be configured for tracking one or more of the trackers (not shown in Fig. 1).

In some variants, at least one tracker with active markers (e.g., light emitting diodes) is provided that emit electromagnetic radiation to be captured by the tracking camera 120. In such or other variants, at least one tracker with passive markers can be provided that reflect electromagnetic radiation to be captured by the tracking camera 120. The reflected electromagnetic radiation may be emitted by a signal generator (e.g., a light source) suitably positioned in the OR.

As shown in Fig. 1, the surgeon 110 may (virtually) be located between the signal detector 120 and the three-dimensional image data 102 (e.g., of a patient anatomy that is to be treated). Therefore, the surgeon 110 may partly block, or shade, a line-of-sight LOS between the signal detector 120 and a tracker (not shown in Fig. 1) that is to be attached to the patient 100. Further, the tracker that is to be attached to the patient 100 may complicate or block the surgeon's 110 access to an anatomical region of interest (not shown). For example, the tracker may block access to the planned incision 104 and/or at least one of the planned trajectories 106 when located between the surgeon 110 and the incision 104 or between the surgeon 110 and one of the trajectories 106.

To optimize the line-of-sight LOS between the signal detector 120 and the tracker that is to be attached to the patient 100 and/or to optimize access to the region of interest, a patient-specific marker arrangement for the tracker is determined based on the planning data. The planning data will in certain embodiments be obtained based on the three-dimensional image data 102 of the patient anatomy and, optionally, the positions of one or more of the surgeon 110, the signal detector 120, the planned incision 104 and one or more of the trajectories of 106 relative to the three-dimensional image data 102.

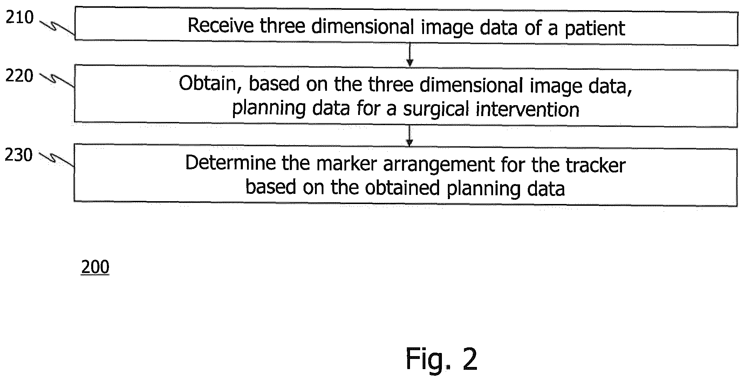

A method for determining a patient-specific marker arrangement for a tracker of a surgical tracking system is described in the following with reference to Figs. 2 to 7F.

Fig. 2 shows a flow diagram of a method 200 for determining a marker arrangement for a tracker of a surgical tracking system based on planning data. The planning data may have been derived as discussed above with reference to Fig. 1 or otherwise.

In a first step 210, three-dimensional image data 102 of a patient 100 is received. As described herein, the image data 102 may be captured via a medical imaging apparatus. The image data 102 may be received from the image apparatus or from a memory, e.g., a database storing the image data 102. In many variants, the three-dimensional image data 102 of the patient 100 will be representative of a patient anatomy, e.g., a patient's spine or one or more vertebrae thereof.

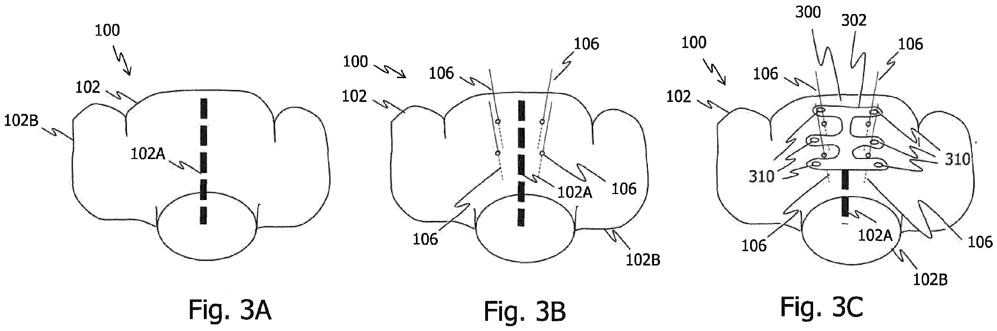

In a second step 220, planning data for a surgical intervention are obtained based on the received image data 102. The planning data may comprise an indication of at least one of i) an anatomical region of interest of a patient 100 (e.g., a certain bone such as a dedicated vertebra or portion thereof), ii) one or more planned incisions 104, iii) one or more planned trajectories 106 for one or more surgical instruments, iv) a planned position and/or orientation of a surgeon 110 relative to the three-dimensional image data 102, v) a planned position and/or orientation of a signal generator and/or a signal detector 120 of a surgical tracking system relative to the three-dimensional image data 102. The planning data may be provided in a coordinate system of the three-dimensional image data 102. Different variants of planning data contents are explained in more detail below with reference to Figs. 3A to 7E.

In a third step 230, a marker arrangement for the tracker of the surgical tracking system is determined based at least in part on the obtained planning data. Different variants for determining the marker arrangement are explained in more detail below, with reference to Figs. 3C, 4B, 5C, 6C and 7E.

Fig. 3A schematically shows the three-dimensional image data 102 of the patient 100. The three-dimensional image data 102 may be determined via image processing of the image data received in the second step 210 of Fig. 2. The anatomical region of interest (not shown) may be determined manually by the surgeon 110 or automatically, for example via image recognition techniques. The three-dimensional image data 102 of the patient 100 in the scenario of Fig. 3A may be representative of at least one of the patient's vertebrae 102A (or any other bone) and/or of a skin surface 102B in a region of these one or more vertebrae 102A. The one or more vertebrae 102A may be in need of a surgical intervention (e.g., placement of one or more pedicle screws.)

Fig. 3B schematically shows planned trajectories 106 of a surgical instrument in the three-dimensional image data 102 of Fig. 3A. The trajectories 106 may be planned preoperatively, for example to define how pedicle screws are to be inserted into a set of vertebrae 102A. The planning data may define an extension of the trajectories 106 in a coordinate system of the three-dimensional image date 102. There exist commercial software tools for such planning procedures.

The trajectories 106 may be planned in a patient-specific manner relative to the three-dimensional image date 102 of the vertebrae 102A. Additionally, or in the alternative, the trajectories 106 may be planned in an indication-specific manner. For example, the trajectories 106 may be planned based at least in part on the three-dimensional image data 102 of the patient 100 and a preferred treatment for the patient 100, e.g., based on the medical history of the patient or a set of patients. The trajectories 106 may further be planned based at least in part on the preferences of the surgeon 110.

Fig. 3C schematically shows a patient tracker 300 virtually superimposed on the three-dimensional image data 102. The exemplary tracker 300 of Fig. 3 is at least partially flexible and virtually attached to the back of the patient 100. The tracker 300 comprises a tracker substrate 302 and multiple markers 310. The multiple markers 310 form a marker arrangement that has been determined based on the three-dimensional image data 102 and the planned trajectories 106 of Fig. 3B (see also step 230 in Fig. 2). The marker arrangement may be defined by Euclidean distances between positions of pairs of markers 310 or by absolute positions in a coordinate system of the tracker 300. This coordinate system may have its origin in one of the markers 310.

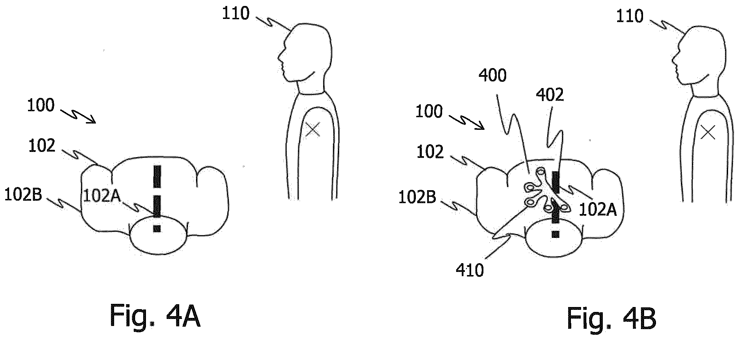

For example, the marker arrangement has been determined based on a rule that each marker 310 must have a minimum distance from each trajectory 106 of the surgical instrument. Additionally, such a rule may also apply to the shape of the tracker substrate 302. The minimum distances may be based at least in part on a margin for correction of the planned trajectories 106. Additionally or alternatively, the markers 310 may have a minimum distance from each other. The minimum distance may be based at least in part on a distance and orientation of the tracker 300 relative to the anatomical region of interest, e.g., one or more vertebrae 102A in which the pedicle screws are to be placed. For example, the larger the distance between the tracker 300 and the anatomical region of interest, the larger the minimum distance between the markers 310 may be defined. One or more of the markers 310 shown in Fig 3C may be virtually arranged relative to at least one of a surface of the patient 100, the planned trajectories 106, the planned incision 104 (not shown in Fig 3C) and the anatomical region of interest (not shown in Fig 3C). Based on such a virtual arrangement, manufacturing (e.g., printing) instructions for manufacturing one or more of the marker arrangement and the tracker substrate 302 may be generated. The above considerations also apply to any of the markers 410, 510, 610, 710 shown in the Figs. 4B, 5C, 6C and 7E described below.

Fig. 4A schematically shows the three-dimensional image data 102 of the patient 100 and a planned position (denoted by an X) of the surgeon 110 relative to the three-dimensional image data 102. This planned surgeon position relative to the three-dimensional image data 102 (e.g., relative to a patient bone such as a vertebra 102A to be treated by the surgeon) is defined in planning data (step 220 of Fig. 2) and forms the basis for determining the marker arrangement (step 230 of Fig. 2).

Fig. 4B schematically shows a tracker 400 superimposed on the three-dimensional image data 102 of Fig. 4A with the associated marker arrangement as determined based on the planning data. As can be seen in Fig. 4B, the marker arrangement is located in a portion (here: the skin surface 102B) of the patient anatomy indicated in three-dimensional image data 102 defined such that a line-of-sight and access from the planned position of the surgeon 110 to the anatomical region of interest is improved, i.e., not blocked by the tracker 400. For example, the markers 410 may be configured to be arrangeable (with the tracker 400) on a side of the patient bone to be treated, that faces away from the surgeon so as to avoid obstruction issues.

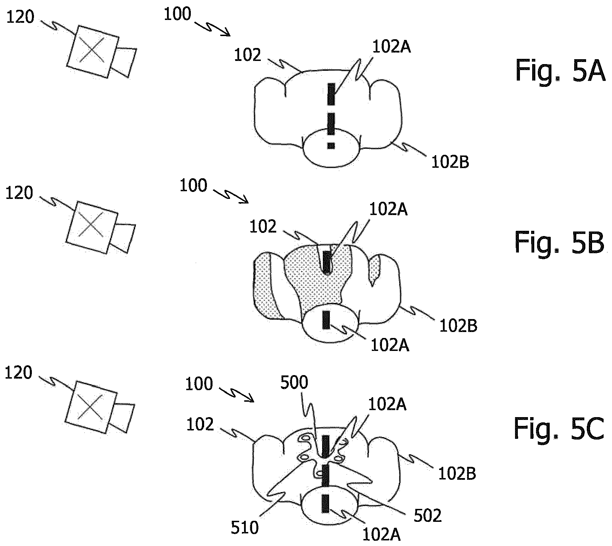

Fig. 5A schematically shows the three-dimensional image data 102 of the patient 100 and a planned position and orientation of the signal detector 120 of the surgical tracking system relative to the three-dimensional image data 102. Based on the location and orientation of the signal detector 120, a line-of-sight of the signal detector 120 may be determined. A part of the three-dimensional image data 102, e.g., a skin surface 102B of the patient 100, is determined as a suitable marker location based on the line-of-sight of the signal detector 120. Non-shaded regions of the skin surface 102B are illustrated in Fig. 5B as dotted regions and define suitable locations for markers (e.g., in the planning data). Shaded regions of the skin surface 102B are no suitable locations for markers since the signal detector 120 has no line-of-sight so such locations. The marker arrangement may thus be determined based on the planning data such that no markers need to be placed in the shaded regions.

Fig. 5C schematically shows a tracker 500 superimposed on the indicated region of Fig. 5B. The tracker 500 comprises a marker arrangement determined based on the line-of-sight of the signal detector of Fig. 5B (i.e., the planning data) so that none of the markers 510 (on the common substrate 502) needs to be placed in a shaded region. In particular, each marker 510 is determined to be located in the non-shaded region of the three-dimensional image data 102.

The size of the markers 510 and/or the distance between the markers 510 may be determined based at least in part on a distance between the signal detector 120 and the three-dimensional image data 102 (e.g., a skin surface 102B of the patient where the markers 510 are eventually to be placed). Larger distances between the signal detector 120 and the three-dimensional image data 102 may require larger sizes of the markers 510 and/or larger distances between the markers 510 (and vice versa).

Further, the marker arrangement may be determined based on an anatomical region of interest of the patient 100. In the example of Fig. 5C, the anatomical region of interest may be a vertebra 102A of the patient 100, and the marker arrangement is thus located on the back of the patient 100 to be located on both sides of the patient's spine but not above one of the vertebrae 102A to be treated. The size of the markers 510 and/or the distance between the markers 510 may be determined based at least in part on a distance between the tracker 500 and the anatomical region of interest (e.g., the distance between the tracker 500 and the vertebrae to be treated). Larger distances between the tracker 500 and the anatomical region of interest may require larger sizes of the markers 510 and/or larger distances between the markers 510 (and vice versa).

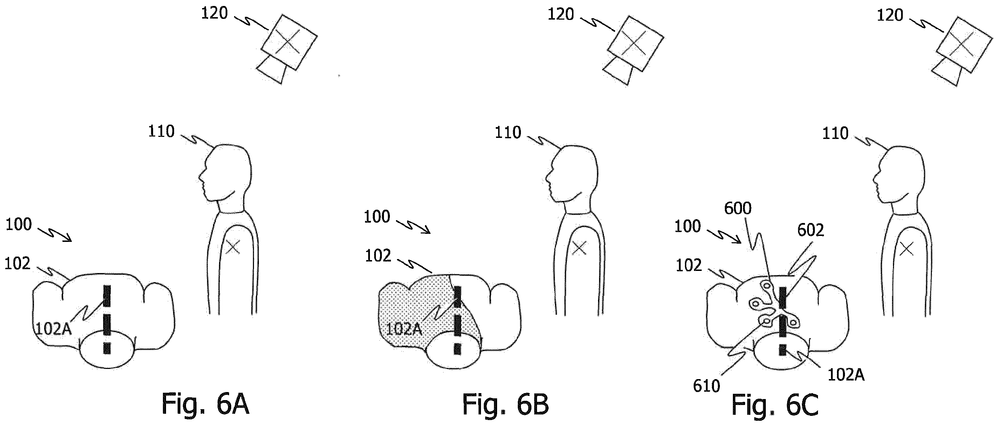

Fig. 6A schematically shows the three-dimensional image data 102 of the patient 100 with planned positions of both the surgeon 110 and the signal detector 120 (as defined in the planning data). Analogously to Fig. 5B, Fig. 6B schematically shows the three-dimensional image data 102 of Fig. 6A with an indication of a region suitable for arranging markers 310, 410, 510, 610, 710 therein, i.e., a non-shaded region in the line-of-sight of the signal detector 120 based on its planned position and orientation relative to the three-dimensional image data 102. In comparison to Fig. 5B, the position of the surgeon 110 relative to the three-dimensional image data 102 (and thus also to the signal detector 120) has also been taken into account for determining the line-of-sight of the signal detector 120 (i.e., shaded locations resulting from the surgeon's planned position). In particular, the line-of-sight from the signal detector 120 to the side of the three-dimensional image data 102 closer to the position of the surgeon 110 is blocked by the surgeon in the example of Fig. 6B.

Fig. 6C schematically shows a tracker 600 superimposed on the indicated region of Fig. 6B. The tracker 600 comprises a marker arrangement determined based on the line-of-sight of the signal detector 120 of Fig. 6B so that the markers 610 on the substrate 602 can be located in non-shaded locations.

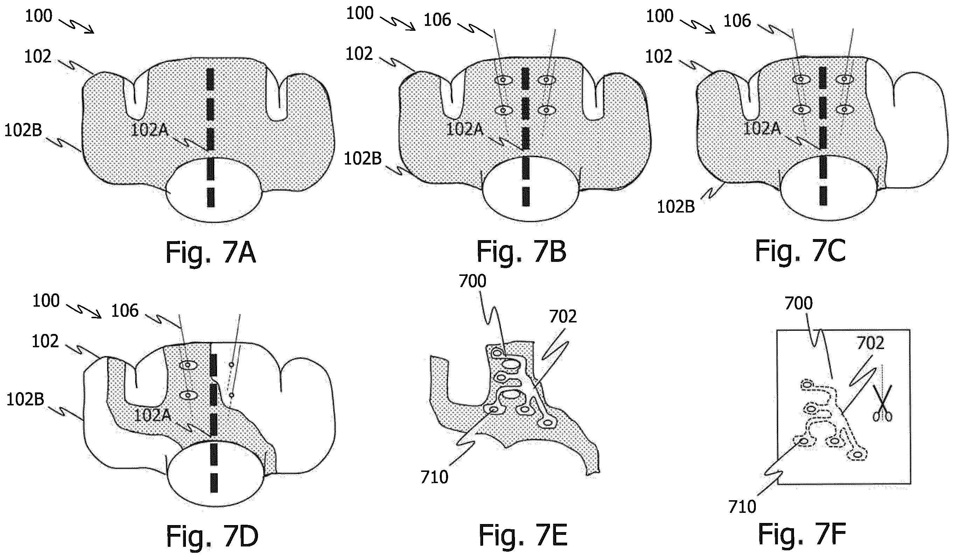

Fig. 7A schematically shows the three-dimensional image data 102 of the patient 100 with an indicated region suitable for arranging markers 310, 410, 510, 610, 710 therein. The indicated region shown in the example of Fig. 7A has been determined based on the three-dimensional image data 102 alone, with no restrictions taken into account.

Fig. 7B schematically shows the three-dimensional image data 102 of Fig. 7A, with the indicated region being restricted based on planned trajectories 106 of the surgical instrument. In this example, an circular area around each of the trajectories 106 is to be kept free, i.e., not indicated as a suitable region for arranging markers 310, 410, 510, 610, 710 therein. The size of the area that is to be kept free may be determined based on at least one of the surgical use-case, patient data, preferences of the surgeon 110, and a margin for correction of the planned trajectories 106. The trajectories 106 relative to the three-dimensional image data 102 may be comprised by the planning data.

Fig. 7C schematically shows the three-dimensional image data 102 of Fig. 7B, with the indicated region being further restricted based on the planned position of the surgeon 110 relative to the three-dimensional image data 102. Analogously to Figs. 4B and 6B, the side of the three-dimensional image data 102 located closer to the position of the surgeon 110 is not suited for a marker position, since the tracker 300, 400, 500, 600, 700 then may block the line-of-sight of the surgeon 110 (or may block access to the anatomical region of interest).

Fig. 7D schematically shows the three-dimensional image data 102 of Fig. 7C, with the indicated region being further restricted based on the planned position and orientation of the signal detector 120. In the example shown in Fig. 7D, analogously to the example shown in Fig. 6B, the line-of-sight of the signal detector 120 is partly blocked by the surgeon 110 and the region of the three-dimensional image data 102 that is thus not in the line-of-sight of the signal detector 120, is not suited as a position for a marker 310, 410, 510, 610, 710.

Fig. 7E schematically shows a tracker 700 virtually superimposed on the indicated region of Fig. 7D. The tracker 700 comprises a marker arrangement 710 determined based on the line-of-sight of the signal detector 120 of Fig. 7D and on the planned trajectories 106.

When determining the marker arrangement, a minimum number of markers 310, 410, 510, 610, 710 comprised by the marker arrangement may be taken into account. This minimum number may be defined in tracker design rules. For example, the minimum number may be 3, 4, 5, 6 or higher.

In some of the above examples, it has been explained how a marker arrangement can be determined in view of certain constraints, with the objective to avoid obstructions for either the surgeon 110 or the signal detector 120. Once the marker arrangement has been determined, it may become necessary to define a suitably shaped tracker substrate 302, 402, 502, 602, 702 configured to accommodate the tracker arrangement. The shape of the tracker substrate 302, 402, 502, 602, 702 may be determined based on the marker arrangement (e.g., so as to accommodate each marker of the marker arrangement). There may exist additional constraints for the substrate 302, 402, 502, 602, 702, such as defining an opening or indentation in locations defined by the trajectories 106 (see Figs. 3C and 7E) or the incision 104 (see Fig. 5C) or relative to an anatomical region of interest (e.g., a patient bone such as a vertebra 102A). Such and further constraints may be defined in tracker design rules).

Once the marker arrangement and, optionally, the shape of the tracker substrate have been determined, instructions may be provided to the surgeon 110 regarding proper placement of the marker arrangement relative to the patient 100 (see Figs. 3C, 4B, 5C, 6C, 7E). The instructions may be visually output relative to the three-dimensional image data of the patient 100 (see, e.g., Fig. 3C)

The method 200 for determining a patient-specific marker arrangement 310, 410, 510, 610, 710 for a tracker 300, 400, 500, 600, 700 of the surgical tracking system described herein may further comprise a step of generating manufacturing instructions for manufacturing the determined marker arrangement 310, 410, 510, 610, 710. In some variants, the manufacturing instructions may comprise at least one of printing instructions and instructions for additive manufacturing. For example, at least a part of the tracker may be manufactured via 3D printing. The 3D-printed tracker may define positions (e.g., comprise support pins) for accommodating the markers (e.g., with the markers being configured as reflecting spheres).

In some variants, the tracker 300, 400, 500, 600, 700, in particular the tracker substrate 302, 402, 502, 602, 702, may comprise a layer stack that may be printed based at least in part on the determined marker arrangement 310, 410, 510, 610, 710 (e.g., to directly print a custom shaped tracker 300, 400, 500, 600, 700). In some variants a standardized layer stack may be printed and the manufacturing instructions may comprise trimming instructions to obtain a custom-shaped tracker 300, 400, 500, 600, 700 with the determined marker arrangement 310, 410, 510, 610, 710 by trimming the printed layer stack.

In the variant shown in Fig. 7F, the manufacturing instructions comprise trimming, e.g., cutting, instructions for a user to indicate how to obtain the custom-shaped tracker 700 with the determined marker arrangement 710 from the substrate 702, e.g., a standardized printed layer stack. In particular, Fig. 7F shows trimming instructions in the form of trimming markings printed on the substrate 702, that indicate where the substrate 702 is to be trimmed to obtain the tracker 300, 400, 500, 600, 700. Alternatively or additionally, the trimming instructions may be provided on a display, e.g., in form of an image of the layer stack with trimming markings or an animation of how to trim the substrate.

As has become apparent from the above description of exemplary embodiments, a technique for determining a patient-specific marker arrangement 310, 410, 510, 610, 710 for a tracker 300, 400, 500, 600, 700 of a surgical tracking system is provided. In some implementations of this technique, the risk of a line-of-sight loss between a signal detector 120 and the tracker 300, 400, 500, 600, 700 can be reduced. As a result, procedures relying on object tracking, such as surgical navigation procedures, become more reliable. In such or other implementations, the risk of obstructing a surgeon 110 can efficiently be reduced, leading to better surgical results.

## Claims

1. A computer-implemented method (200) for determining a patient-specific marker arrangement (310, 410, 510, 610, 710) for a tracker (300, 400, 500, 600, 700) of a surgical tracking system, wherein the marker arrangement is defined by marker positions, the method comprising
receiving (210) three-dimensional image data (102) of a patient;
obtaining (220), based on the three-dimensional image data, planning data for a surgical intervention; and
determining (230) the marker arrangement for the tracker based at least on the obtained planning data;
**characterized in that** the planning data also are indicative of a planned position of a signal generator or signal detector (120) of the surgical tracking system relative to a patient anatomy represented in the three dimensional image data (102).

2. The computer-implemented method (200) according to claim 1, further comprising
generating manufacturing instructions for manufacturing the marker arrangement (310, 410, 510, 610, 710) as determined based at least on the obtained planning data.

3. The computer-implemented method (200) according to claim 2, wherein
the manufacturing instructions comprise at least one of printing instructions and instructions for additive manufacturing.

4. The computer-implemented method (200) according to any of the preceding claims, further comprising
determining a surface of the patient (100) based on the three-dimensional image data (102), wherein the marker arrangement (310, 410, 510, 610, 710) is determined also based on the determined surface of the patient (100).

5. The computer-implemented method (200) according to claim 4, wherein
determining (230) the marker arrangement (310, 410, 510, 610, 710) comprises virtually arranging one or more markers on the determined surface of the patient (100).

6. The computer-implemented method (200) according to any of the preceding claims, wherein
the planning data are indicative of an anatomical region of interest of the patient (100).

7. The computer-implemented method (200) according to claim 6, wherein
determining (230) the marker arrangement (310, 410, 510, 610, 710) comprises virtually arranging one or more markers (310, 410, 510, 610, 710) relative to the anatomical region of interest.

8. The computer-implemented method (200) according to any of the preceding claims, wherein
the planning data are indicative of a planned trajectory (106) of a surgical instrument.

9. The computer-implemented method (200) according to claim 8, wherein
determining (230) the marker arrangement (310, 410, 510, 610, 710) comprises virtually arranging one or more markers (310, 410, 510, 610, 710) relative to the planned trajectory (106).

10. The computer-implemented method (200) according to any of the preceding claims, wherein
the planning data are indicative of a planned incision (104).

11. The computer-implemented method (200) according to claim 10, wherein
determining (230) the marker arrangement (310, 410, 510, 610, 710) comprises virtually arranging one or more markers (310, 410, 510, 610, 710) relative to the planned incision (104).

12. The computer-implemented method (200) according to any of the preceding claims, further comprising
determining a region of the three-dimensional image data (102) that has a line-of-sight to the signal generator or signal detector (120) based on the planning data, wherein the marker arrangement (310, 410, 510, 610, 710) is determined based on the determined region of the three-dimensional image data.

13. The computer-implemented method (200) according to any of the preceding claims, wherein the tracker (300, 400, 500, 600, 700) comprises a substrate (302, 402, 502, 602, 702) supporting the marker arrangement (310, 410, 510, 610, 710), the method further comprising
determining a shape of the substrate based on the planning data.

14. The computer-implemented method (200) according to any of the preceding claims, wherein the three-dimensional image data (102) have been captured by an imaging apparatus, the method further comprising
receiving positional data indicative of a position of the imaging apparatus when capturing the three-dimensional image data, wherein the marker arrangement (310, 410, 510, 610, 710) is determined based on the positional data.

15. The computer-implemented method (200) according to any of the preceding claims, wherein
determining (230) the marker arrangement (310, 410, 510, 610, 710) takes into account a criterion related to a minimum distance between markers.

16. A computer program product, comprising instructions that, when executed on at least one processor, cause the at least one processor to carry out any of the methods (200) of claims 1 to 15.

17. An apparatus for determining a patient-specific marker arrangement (310, 410, 510, 610, 710) for a tracker (300, 400, 500, 600, 700) of a surgical tracking system, wherein the marker arrangement is defined by marker positions, wherein the apparatus is configured to
receive (210) three-dimensional image data (102) of a patient (100);
obtain (220), based on the three-dimensional image data, planning data for a surgical intervention; and
determine (230) the marker arrangement for the tracker based at least on the obtained planning data;
**characterized in that** the planning data also are indicative of a planned position of a signal generator or signal detector (120) of the surgical tracking system relative to a patient anatomy represented in the three dimensional image data (102).

18. The apparatus of claim 17, configured to perform any of the method steps of claims 2 to 15.

19. A system comprising
the apparatus according to claim 17 or 18; and
a manufacturing device configured to manufacture a tracker (300, 400, 500, 600, 700) with the determined marker arrangement (310, 410, 510, 610, 710).

## Patentansprüche

1. Computerimplementiertes Verfahren (200) zum Festlegen einer patientenspezifischen Markeranordnung (310, 410, 510, 610, 710) für einen Verfolger (300, 400, 500, 600, 700) eines chirurgischen Verfolgungssystems, wobei die Markeranordnung durch Markerpositionen definiert ist, wobei das Verfahren umfasst
Empfangen (210) dreidimensionaler Bilddaten (102) eines Patienten;
Erhalten (220) von Planungsdaten für einen chirurgischen Eingriff auf der Grundlage der dreidimensionalen Bilddaten; und
Festlegen (230) der Markeranordnung für den Verfolger zumindest auf der Grundlage der erhaltenen Planungsdaten,
**dadurch gekennzeichnet, dass** die Planungsdaten auch eine geplante Position eines Signalgenerators oder Signaldetektors (120) des chirurgischen Verfolgungssystems relativ zu einer in den dreidimensionalen Bilddaten (102) dargestellten Patientenanatomie angeben.

2. Computerimplementiertes Verfahren (200) nach Anspruch 1, ferner umfassend
Erzeugen von Fertigungsanweisungen zum Fertigen der Markeranordnung (310, 410, 510, 610, 710), wie zumindest auf der Grundlage der erhaltenen Planungsdaten bestimmt.

3. Computerimplementiertes Verfahren (200) nach Anspruch 2, wobei
die Fertigungsanweisungen mindestens eine der folgenden Anweisungen umfassen: Druckanweisungen und Anweisungen für additives Fertigen.

4. Computerimplementiertes Verfahren (200) nach einem der vorhergehenden Ansprüche, ferner umfassend
Bestimmen einer Oberfläche des Patienten (100) auf der Grundlage der dreidimensionalen Bilddaten (102), wobei die Markeranordnung (310, 410, 510, 610, 710) auch auf der Grundlage der ermittelten Oberfläche des Patienten (100) festgelegt wird.

5. Computerimplementiertes Verfahren (200) nach Anspruch 4, wobei
das Festlegen (230) der Markeranordnung (310, 410, 510, 610, 710) ein virtuelles Anordnen eines oder mehrerer Marker auf der bestimmten Oberfläche des Patienten (100) umfasst.

6. Computerimplementiertes Verfahren (200) gemäß einem der vorhergehenden Ansprüche, wobei
die Planungsdaten einen anatomischen Bereich des Patienten (100) angeben, der von Interesse ist.

7. Computerimplementiertes Verfahren (200) gemäß Anspruch 6, wobei
das Festlegen (230) der Markeranordnung (310, 410, 510, 610, 710) das virtuelle Anordnen eines oder mehrerer Marker (310, 410, 510, 610, 710) relativ zu dem anatomischen Bereich von Interesse umfasst.

8. Computerimplementiertes Verfahren (200) nach einem der vorhergehenden Ansprüche, wobei
die Planungsdaten eine geplante Trajektorie (106) eines chirurgischen Instruments angeben.

9. Computerimplementiertes Verfahren (200) nach Anspruch 8, wobei
das Festlegen (230) der Markeranordnung (310, 410, 510, 610, 710) das virtuelle Anordnen eines oder mehrerer Marker (310, 410, 510, 610, 710) relativ zu der geplanten Trajektorie (106) umfasst.

10. Computerimplementiertes Verfahren (200) nach einem der vorhergehenden Ansprüche, wobei
die Planungsdaten einen geplanten Schnitt (104) angeben.

11. Computerimplementiertes Verfahren (200) nach Anspruch 10, wobei
das Festlegen (230) der Markeranordnung (310, 410, 510, 610, 710) das virtuelle Anordnen eines oder mehrerer Marker (310, 410, 510, 610, 710) relativ zu dem geplanten Schnitt (104) umfasst.

12. Computerimplementiertes Verfahren (200) nach einem der vorhergehenden Ansprüche, ferner umfassend
Bestimmen eines Bereichs der dreidimensionalen Bilddaten (102), der eine Sichtlinie zum Signalgenerator oder Signaldetektor (120) aufweist, basierend auf den Planungsdaten, wobei die Markeranordnung (310, 410, 510, 610, 710) basierend auf dem bestimmten Bereich der dreidimensionalen Bilddaten festgelegt wird.

13. Computerimplementiertes Verfahren (200) gemäß einem der vorhergehenden Ansprüche, wobei der Verfolger (300, 400, 500, 600, 700) ein Substrat (302, 402, 502, 602, 702) umfasst, das die Markeranordnung (310, 410, 510, 610, 710) trägt, wobei das Verfahren ferner umfasst:
Festlegen einer Form des Substrats auf der Grundlage der Planungsdaten.

14. Computerimplementiertes Verfahren (200) nach einem der vorhergehenden Ansprüche, wobei die dreidimensionalen Bilddaten (102) von einer Bildgebungsvorrichtung erfasst worden sind, wobei das Verfahren ferner umfasst:
Empfangen von Positionsdaten, die eine Position der Bildgebungsvorrichtung beim Erfassen der dreidimensionalen Bilddaten angeben, wobei die Markeranordnung (310, 410, 510, 610, 710) auf der Grundlage der Positionsdaten festgelegt wird.

15. Computerimplementiertes Verfahren (200) nach einem der vorhergehenden Ansprüche, wobei
das Festlegen (230) der Markeranordnung (310, 410, 510, 610, 710) ein Kriterium berücksichtigt, das sich auf einen Mindestabstand zwischen Markern bezieht.

16. Computerprogrammprodukt, das Anweisungen umfasst, die, wenn sie auf mindestens einem Prozessor ausgeführt werden, den mindestens einen Prozessor veranlassen, eines der Verfahren (200) der Ansprüche 1 bis 15 auszuführen.

17. Vorrichtung zur Festlegung einer patientenspezifischen Markeranordnung (310, 410, 510, 610, 710) für einen Verfolger (300, 400, 500, 600, 700) eines chirurgischen Verfolgungssystems, wobei die Markeranordnung durch Markerpositionen definiert ist, wobei die Vorrichtung ausgebildet ist, um
dreidimensionale Bilddaten (102) eines Patienten (100) zu empfangen (210);
auf der Grundlage der dreidimensionalen Bilddaten Planungsdaten für einen chirurgischen Eingriff zu erhalten (220) und
die Markeranordnung für den Verfolger zumindest auf der Grundlage der erhaltenen Planungsdaten festzulegen (230);
**dadurch gekennzeichnet, dass** die Planungsdaten auch eine geplante Position eines Signalgenerators oder Signaldetektors (120) des chirurgischen Verfolgungssystems relativ zu einer in den dreidimensionalen Bilddaten (102) dargestellten Patientenanatomie angeben.

18. Vorrichtung nach Anspruch 17, ausgebildet, um einen beliebigen der Verfahrensschritte der Ansprüche 2 bis 15 auszuführen.

19. System, umfassend
die Vorrichtung gemäß Anspruch 17 oder 18; und
eine Fertigungsvorrichtung, die ausgebildet ist, um einen Verfolger (300, 400, 500, 600, 700) mit der festgelegten Markeranordnung (310, 410, 510, 610, 710) herzustellen.

## Revendications

1. Procédé mis en œuvre par ordinateur (200) destiné à déterminer un agencement de marqueurs spécifique au patient (310, 410, 510, 610, 710) pour un dispositif de suivi (300, 400, 500, 600, 700) d'un système de suivi chirurgical, dans lequel l'agencement de marqueurs est défini par des positions de marqueurs, le procédé comprenant
la réception (210) de données d'images tridimensionnelles (102) d'un patient;
l'obtention (220), sur la base des données d'images tridimensionnelles, de données de planification pour une intervention chirurgicale, et
la détermination (230) de l'agencement de marqueurs pour le suiveur sur la base au moins des données de planification obtenues, **caractérisé en ce que** les données de planification indiquent également une position planifiée d'un générateur de signaux ou d'un détecteur de signaux (120) du système de suivi chirurgical par rapport à l'anatomie du patient représentée dans les données d'image tridimensionnelles (102).

2. Procédé mis en œuvre par ordinateur (200) selon la revendication 1, comprenant en outre
la génération d'instructions de fabrication pour la fabrication de l'agencement de marqueurs (310, 410, 510, 610, 710) déterminé au moins sur la base des données de planification obtenues.

3. Procédé mis en œuvre par ordinateur (200) selon la revendication 2, dans lequel
les instructions de fabrication comprennent au moins l'une parmi des instructions d'impression et des instructions de fabrication additive.

4. Procédé mis en œuvre par ordinateur (200) selon l'une quelconque des revendications précédentes, comprenant en outre
la détermination d'une zone du patient (100) sur la base des données d'image tridimensionnelles (102), l'agencement de marqueurs (310, 410, 510, 610, 710) étant également déterminé sur la base de la zone déterminée du patient (100).

5. Procédé mis en œuvre par ordinateur (200) selon la revendication 4, dans lequel
la détermination (230) de l'agencement de marqueur (310, 410, 510, 610, 710) comprend la disposition virtuelle d'un ou plusieurs marqueurs sur la zone déterminée du patient (100).

6. Procédé mis en œuvre par ordinateur (200) selon l'une quelconque des revendications précédentes, dans lequel
les données de planification indiquent une région anatomique d'intérêt du patient (100).

7. Procédé mis en œuvre par ordinateur (200) selon la revendication 6, dans lequel
la détermination (230) de l'agencement de marqueurs (310, 410, 510, 610, 710) comprend la disposition virtuelle d'un ou de plusieurs marqueurs (310, 410, 510, 610, 710) par rapport à la région anatomique d'intérêt.

8. Procédé mis en œuvre par ordinateur (200) selon l'une quelconque des revendications précédentes, dans lequel
les données de planification indiquent une trajectoire planifiée (106) d'un instrument chirurgical.

9. Procédé mis en œuvre par ordinateur (200) selon la revendication 8, dans lequel
la détermination (230) de l'agencement de marqueurs (310, 410, 510, 610, 710) comprend la disposition virtuelle d'un ou de plusieurs marqueurs (310, 410, 510, 610, 710) par rapport à la trajectoire planifiée (106).

10. Procédé mis en œuvre par ordinateur (200) selon l'une quelconque des revendications précédentes, dans lequel
les données de planification indiquent une incision planifiée (104).

11. Procédé mis en œuvre par ordinateur (200) selon la revendication 10, dans lequel
la détermination (230) de l'agencement de marqueurs (310, 410, 510, 610, 710) comprend la disposition virtuelle d'un ou de plusieurs marqueurs (310, 410, 510, 610, 710) par rapport à l'incision planifiée (104).

12. Procédé mis en œuvre par ordinateur (200) selon l'une quelconque des revendications précédentes, comprenant en outre
la détermination d'une région des données d'image tridimensionnelles (102) qui a une ligne de visée vers le générateur de signaux ou le détecteur de signaux (120) sur la base des données de planification, l'agencement de marqueurs (310, 410, 510, 610, 710) étant déterminé sur la base de la région déterminée des données d'image tridimensionnelles.

13. Procédé mis en œuvre par ordinateur (200) selon l'une quelconque des revendications précédentes, dans lequel le suiveur (300, 400, 500, 600, 700) comprend un substrat (302, 402, 502, 602, 702) supportant l'agencement de marqueurs (310, 410, 510, 610, 710), le procédé comprenant en outre
la détermination de la forme du substrat sur la base des données de planification.

14. Procédé mis en œuvre par ordinateur (200) selon l'une quelconque des revendications précédentes, dans lequel les données d'image tridimensionnelle (102) ont été capturées par un appareil d'imagerie, le procédé comprenant en outre
la réception de données de position indiquant la position de l'appareil d'imagerie lors de la capture des données d'image tridimensionnelles, l'agencement des marqueurs (310, 410, 510, 610, 710) étant déterminé sur la base des données de position.

15. Procédé mis en œuvre par ordinateur (200) selon l'une quelconque des revendications précédentes, dans lequel
la détermination (230) de l'agencement des marqueurs (310, 410, 510, 610, 710) tient compte d'un critère relatif à une distance minimale entre les marqueurs.

16. Produit programme informatique, comprenant des instructions qui, lorsqu'elles sont exécutées sur au moins un processeur, amènent l'au moins un processeur à mettre en œuvre l'un des procédés (200) des revendications 1 à 15.

17. Appareil permettant de déterminer un agencement de marqueurs spécifique au patient (310, 410, 510, 610, 710) pour un dispositif de suivi (300, 400, 500, 600, 700) d'un système de suivi chirurgical, dans lequel l'agencement de marqueurs est défini par des positions de marqueurs, l'appareil étant conçu pour
recevoir (210) des données d'images tridimensionnelles (102) d'un patient(100);
obtenir (220), sur la base des données d'images tridimensionnelles, des données de planification pour une intervention chirurgicale; et
déterminer (230) l'agencement de marqueurs pour le suiveur sur la base au moins des données de planification obtenues, **caractérisé en ce que** les données de planification indiquent également une position planifiée d'un générateur de signaux ou d'un détecteur de signaux (120) du système de suivi chirurgical par rapport à l'anatomie du patient représentée dans les données d'image tridimensionnelles (102).

18. Appareil selon la revendication 17, configuré pour effectuer l'une quelconque des étapes du procédé selon les revendications 2 à 15.

19. Système comprenant
l'appareil selon la revendication 17 ou 18; et
un dispositif de fabrication conçu pour fabriquer un suiveur (300, 400, 500, 600, 700) à l'aide de l'agencement de marqueurs déterminé (310, 410, 510, 610, 710).
